# EUROPÄISCHE PATENTSCHRIFT

(11) **EP 1 769 814 B1**
(45) Veröffentlichungstag und Bekanntmachung des Hinweises auf die Patenterteilung: **16.04.2008**
(21) Anmeldenummer: 05021508.6
(22) Anmeldetag: 30.09.2005
(51) Int. Cl.: A61M 5/145, A61M 5/315

(54) **Antriebsvorrichtung für einen Kolben in einem ein flüssiges Produkt enthaltenden Behältnis**
Driver for a plunger in a container holding a liquid product
Dispositif d'actionnement pour un piston situé dans un contenant renfermant un liquide

(43) Veröffentlichungstag der Anmeldung: 04.04.2007
(73) Patentinhaber: F. Hoffmann-La Roche AG, 4070 Basel (CH); Roche Diagnostics GmbH, 68305 Mannheim (DE)
(72) Erfinder: Harttig, Herbert, 67434 Neustadt (DE)
(74) Vertreter: Isenbruck, Günter

(56) Entgegenhaltungen:
- WO-A-01/72358
- WO-A-03/103763
- US-A- 5 222 362

## Beschreibung

Die Erfindung bezieht sich auf eine Antriebsvorrichtung, die einen Kolben in einem Behältnis antreibt, wobei das Behältnis ein flüssiges Produkt enthält, das durch den Kolben über einen Auslass aus dem Behältnis herausgetrieben wird. Solche Antriebsvorrichtungen können insbesondere zur Dosierung eines injizierbaren flüssigen Produkts (zum Beispiel Insulin) in einem Infusionssystem Verwendung finden.

Die kontinuierliche Infusion von Medikamenten beziehungsweise die diskontinuierliche, gesteuerte Infusion, wie sie zum Beispiel bei der Verabreichung von Insulin notwendig ist, erfolgt in der Regel über sogenannte Spritzenpumpen. Dabei wird der Kolben einer Spritze durch einen geeigneten Stempel kontinuierlich oder diskontinuierlich nach vorne geschoben und ein Medikament dem Patienten über eine Schlauchverbindung und eine Injektionskanüle verabreicht.

Bei Patienten mit Diabetes bedeutet die Verwendung einer Pumpe zur Applikation von Insulin eine wesentliche Erleichterung im täglichen Umgang mit ihrer Krankheit. Dabei ist eine wichtige Anforderung an eine solche Insulinpumpe eine möglichst geringe Größe und damit verbunden ein möglichst geringes Gewicht. Präzision, Zuverlässigkeit und einfache Bedienung werden gemeinhin vorausgesetzt.

Nachteilig an Pumpen gemäß dem Stand der Technik sind häufig ihre Größe, ihr Gewicht und ihre hohen Herstellkosten, bedingt durch die aufwendige Antriebsmechanik.

Aus der WO 01/72358 A1 ist eine Antriebsvorrichtung für einen Kolben in einem ein injizierbares Produkt enthaltenden Behältnis bekannt. Diese Antriebsvorrichtung enthält mehrere Verschiebestufen und einen Antrieb zum Vorschieben der Verschiebestufen, der über Spindeltriebe auf die Verschiebestufen wirkt. Ein solcher Teleskop-Antrieb erfordert allerdings einen hohen Aufwand für den Antrieb mit Motor und Getriebe und für die dazugehörige Steuerung.

US 6,723,072 B2 bezieht sich auf eine Vorrichtung zum Verabreichen eines flüssigen Medikaments mit einer Kolbenanordnung, die ein Formgedächtniselement enthält, das zwischen zwei Segmenten angeordnet ist und das seine Länge ändern kann. Durch ein abwechselndes Verlängern und Verkürzen des Formgedächtniselements wird dabei eine longitudinale Bewegung der Kolbenanordnung hervorgerufen, da die zwei Segmente im Wesentlichen nur in einer Richtung verschiebbar sind. Dabei wird nicht deutlich, wodurch eine Bewegung der Segmente in die andere Richtung verhindert wird. Der erreichbare Druck des Kolbens ist durch diese Ausgestaltung stark limitiert. Bei leichten Variationen im Innendurchmesser der Ampulle kann ein solch einfaches System versagen, da die Differenzen in der Haftreibung zwischen den beiden Segmenten zu Null werden können.

Die Aufgabe der vorliegenden Erfindung ist es, die Nachteile des Standes der Technik zu vermeiden. Insbesondere soll eine Antriebsvorrichtung für einen Kolben bereitgestellt werden, die eine geringe Größe, ein geringes Gewicht und geringe Herstellungskosten aufweist.

Diese Aufgabe wird erfindungsgemäß gelöst durch eine Antriebsvorrichtung für einen Kolben in einem ein flüssiges Produkt enthaltenden Behältnis, wobei das Behältnis begrenzende Seitenwände aufweist, die sich in Längsrichtung zu einem Auslass für das flüssige Produkt hin erstrecken. Die erfindungsgemäße Antriebsvorrichtung enthält ein erstes, dem Auslass zugewandtes Segment mit einer aktiv veränderbaren Form, das in einer ersten Form zwischen den Seitenwänden des Behältnisses verklemmt ist und in einer zweiten Form von den Seitenwänden weitgehend gelöst ist. Ferner enthält die Antriebsvorrichtung ein zweites, von dem Auslass abgewandtes Segment mit einer aktiv veränderbaren Form, das in einer ersten Form zwischen den Seitenwänden des Behältnisses verklemmt ist und in einer zweiten Form von den Seitenwänden weitgehend gelöst ist. Ein Verbindungssegment ist zwischen dem ersten und dem zweiten Segment angeordnet und verbindet das erste und zweite Segment miteinander. Das Verbindungssegment weist eine aktiv veränderbare Verbindungslänge auf.

Die erfindungsgemäße Antriebsvorrichtung ist in dem Behältnis angeordnet, in dem sich auch das flüssige Produkt befindet, wobei der Kolben die Antriebsvorrichtung von dem flüssigen Produkt trennt. Ein großer Vorteil der erfindungsgemäßen Lösung liegt in der geringen Größe des Antriebs. Durch die Position des Antriebs im Inneren des Behältnisses wird ein ansonsten nicht nutzbarer Raum verwendet. Durch die besondere Konstruktion entfallen aufwendige Getriebe und Motoren, wodurch Gewicht und Herstellungskosten gespart werden können.

Der Auslass des Behältnisses kann sich bei der erfindungsgemäßen Antriebsvorrichtung zum Beispiel in den Seitenwänden des Behältnisses befinden oder an seinem Ende senkrecht zu den Seitenwänden angeordnet sein.

Das erste und das zweite Segment der erfindungsgemäßen Antriebsvorrichtung weisen eine aktiv veränderbare Form auf. Aktiv veränderbar heißt in diesem Zusammenhang, dass die Formänderung durch äußere Eingriffe (zum Beispiel durch Anlegen eines elektrischen Stroms, Wärmezufuhr oder -ableitung etc.) gezielt hervorgerufen und gesteuert wird. Die Formänderung ist dabei reversibel. Die Formänderung ist gegebenenfalls mit einer Volumenänderung des jeweiligen Segments verbunden.

Wenn das jeweilige erste oder zweite Segment seine erste Form einnimmt, ist es zwischen den Seitenwänden des Behältnisses verklemmt. Dabei besteht zwischen dem Segment und den Seitenwänden vorzugsweise eine Reibkraftverbindung, die ein Verschieben des Segments in dem Behältnis nicht zulässt.

Wenn das jeweilige erste oder zweite Segment seine zweite Form einnimmt, ist es von den Seitenwänden weitgehend gelöst, so dass es in dem Behälter (insbesondere in Längsrichtung zu dem Auslass des Behältnisses hin) verschiebbar ist.

Die beiden Segmente sind durch das Verbindungselement miteinander verbunden, das eine aktiv veränderbare Länge aufweist. Somit wird der Abstand zwischen den beiden Segmenten durch eine Längenänderung des Verbindungselements verändert. Die Längenänderung ist reversibel. Aktiv veränderbar heißt in diesem Zusammenhang, dass die Längenänderung durch äußere Eingriffe (zum Beispiel durch Anlegen eines elektrischen Stroms, Wärmezufuhr oder -ableitung etc.) gezielt hervorgerufen und gesteuert wird. Die Längenänderung kann insbesondere mit einer Volumenänderung des Verbindungselements verknüpft sein.

Mit der erfindungsgemäßen Antriebsvorrichtung wird das flüssige Produkt aus dem Behältnis heraus dosiert, indem die Antriebsvorrichtung den Kolben in dem Behältnis zu dem Auslass hin verschiebt und so das flüssige Produkt durch den Auslass herausdrückt.

Um ein solches Verschieben des Kolbens zu erreichen, führen die beiden Segmente und das Verbindungssegment den folgenden Bewegungsablauf durch:
- Verklemmen des zweiten Segments zwischen den Seitenwänden durch eine aktive Formänderung des zweiten Segments,
- Lösen des ersten Segments von den Seitenwänden durch eine aktive Formänderung des ersten Segments,
- Vergrößern der Länge des Verbindungssegments, wobei sich das erste Segment in Richtung des Auslasses verschiebt,
- Verklemmen des ersten Segments zwischen den Seitenwänden durch eine aktive Formänderung des ersten Segments,
- Lösen des zweiten Segments von den Seitenwänden durch eine aktive Formänderung des zweiten Segments und
- Verkleinern der Länge des Verbindungssegments, wobei sich das zweite Segment in Richtung des Auslasses verschiebt.

Das erste Segment hat direkten Kontakt zu dem Kolben und schiebt diesen bei seiner Bewegung auf den Auslass des Behältnisses zu. Vorzugsweise ist das erste Segment mit dem Kolben fest verbunden oder einstückig mit dem Kolben ausgebildet. Dadurch wird zum Beispiel vermieden, dass sich der Kolben ohne die Antriebsvorrichtung bewegt und flüssiges Produkt (zum Beispiel durch einen Unterdruck am Auslass des Behältnisses) ungewollt austreten kann. Die feste (gegebenenfalls lösbare) Verbindung zwischen dem Kolben und dem ersten Segment der erfindungsgemäßen Antriebsvorrichtung kann zum Beispiel durch eine Schnappverbindung, durch eine Magnetkupplung oder mit einem geeigneten Aktuator erfolgen.

Gemäß einer bevorzugten Ausführungsform der vorliegenden Erfindung umfassen das erste und das zweite Segment erste beziehungsweise zweite Aktuatoren zur aktiven Veränderung der Form des ersten beziehungsweise des zweiten Segments. Ferner ist vorzugsweise auch das Verbindungselement selbst als Aktuator ausgeführt oder es umfasst einen Aktuator, um die Längenänderung des Verbindungselements aktiv zu erreichen. Die Aktuatoren sind dabei ansteuerbar, insbesondere durch Strom oder Wärme, so dass bei der Ansteuerung (Aktivierung) des jeweiligen Aktuators eine Formänderung des jeweiligen Segments beziehungsweise eine Längenänderung des Verbindungselements erfolgt. Der Aktuator weist bei seiner Aktivierung zum Beispiel eine Volumenvergrößerung auf, die beispielsweise durch Wärmedehnung oder eine Phasenänderung hervorgerufen wird, durch die eine Kraft zumindest auf Abschnitte des Segments ausgeübt wird, die ein Verklemmen oder Lösen des Segments von den Seitenwänden des Behältnisses hervorruft. Das gesamte Segment selbst kann in einer Ausführungsform der vorliegenden Erfindung ein Aktuator sein.

Als Aktuatoren können solche eingesetzt werden, die den räumlichen Anforderungen gerecht werden, unabhängig von dem zugrundeliegenden Prinzip. Denkbar sind elektromagnetische, piezoelektrische, chemische, elektrochemische, thermische oder sonstige Aktuatoren. Ein Beispiel für verwendbare Aktuatoren sind auch die in US 6,723,072 B2 genannten Formgedächtnislegierungen. Gemäß einer bevorzugten Ausfiihrungsform der vorliegenden Erfindung sind die Aktuatoren Paraffinaktuatoren, wobei die Antriebsvorrichtung Heizelemente zum Erwärmen der Paraffinaktuatoren umfasst. Solche Aktuatoren werden zum Beispiel in Carlen, Mastrangelo: "Simple, high actuation power, thermally activated paraffin microactuator", vorgestellt bei der Transducers 1999 Conference, Sendai, Japan, 7. bis 10. Juni 1999 beschrieben. Die Paraffinaktuatoren weisen zum Beispiel gegenüber den Formgedächtnislegierungen Vorteile auf. Die maximale Längenänderung der Formgedächtnislegierungen beträgt nur 5%. Damit sind größere Bauteile oder aufwendigere Hebelsysteme notwendig, um die notwendigen Aktuationslängen zu erreichen. Diese Längen dürfen einen Mindestbetrag nicht unterschreiten, da die elastische Verformung des Kolbens überschritten werden muss, bevor eine Relativbewegung zwischen Kolben und Behältniswand erfolgt. Paraffinaktuatoren ermöglichen mit einer Volumenexpansion von > 15 % eine höhere lineare Ausdehnung und erlauben somit eine kleinere Bauweise. Durch die Arbeitstemperatur im Bereich von 60 °C sind ferner die thermischen Verluste deutlich kleiner. Die Temperaturen zum Phasenwechsel bei Formgedächtnislegierungen liegen in der Nähe von 100 °C. Dies bedingt einen höheren Wärmeverlust und damit einen geringeren Wirkungsgrad des Antriebs.

Gemäß einer bevorzugten Ausführungsform der vorliegenden Erfindung ist das erste Segment so ausgebildet, dass es bei einem nicht aktivierten ersten Aktuator in der zweiten Form vorliegt und das zweite Segment ist so ausgebildet, dass es bei nicht aktiviertem zweiten Aktuator in der ersten Form vorliegt. Bei inaktivem ersten Aktuator ist das erste Segment also von den Seitenwänden des Behältnisses gelöst, während das zweite Segment bei inaktivem zweiten Aktuator zwischen den Seitenwänden des Behältnisses verklemmt ist. Daher ist der Antrieb, wenn alle Aktuatoren deaktiviert sind und keine Energie verbraucht wird, trotzdem zwischen den Seitenwänden des Behältnisses verklemmt und ohne eine gezielte Aktivierung der Aktuatoren nicht verschiebbar.

Gemäß einer Ausführungsform der vorliegenden Erfindung ist das Behältnis eine Ampulle mit einem kreisförmigen Querschnitt und die ersten und zweiten Segmente umfassen Spannringe, die mit Hilfe jeweils eines Aktuators zwischen einer inneren Wandung der Ampulle verklemmbar oder von dieser lösbar sind. Dabei weisen die Spannringe vorzugsweise jeweils einen Ringdurchmesser auf, der durch ein Aktivieren der Aktuatoren veränderbar ist. Die Spannringe selbst können Aktuatoren sein, zum Beispiel Bimetallringe, die sich je nach Aufbau bei der Aktivierung öffnen und verengen. In diesem Fall sind keine zusätzlichen Aktuatoren für die Segmente notwendig.

Gemäß einer bevorzugten Ausfiihrungsform der vorliegenden Erfindung ist das erste Segment als offener Ring mit zwei in den Ring hineinragenden Zungen ausgebildet, wobei zwischen den beiden Zungen ein Aktuator angeordnet ist, dessen Dicke aktiv veränderbar ist. Ferner kann das zweite Segment als offener Ring mit zwei in den Ring hineinragenden Zungen ausgebildet sein, wobei eine der Zungen und ein neben der anderen Zunge angeordnetes Widerlager mit einer Grundplatte verbunden sind und zwischen der anderen Zunge und dem Widerlager ein Aktuator angeordnet ist, dessen Dicke aktiv veränderbar ist. Durch die Befestigung der einen Zunge und des Widerlagers an der Grundplatte wird sichergestellt, dass eine feste räumliche Beziehung zwischen der einen Zunge und dem Widerlager besteht.

Zur Kontrolle der Position der erfindungsgemäßen Antriebsvorrichtung oder des Kolbens in dem Behältnis kann ein Messsystem, zum Beispiel auf der Basis der Lasertriangulation, eingesetzt werden. Damit ist es möglich, mit Hilfe geeigneter elektronischer Prozessoren die exakte Position des Kolbens in dem Behältnis zu bestimmen. Daraus lässt sich die dosierte Menge an flüssigem Produkt ableiten und die Schrittweite des nächsten Zyklus an die gewünschte Dosierung anpassen.

Die vorliegende Erfindung hat weiterhin die Verwendung der erfindungsgemäßen Antriebsvorrichtung zum Dosieren eines flüssigen Produkts in einem Infusionssystem zum Gegenstand. Die Antriebsvorrichtung kann insbesondere in einer Pumpe zur Applikation von Insulin aus einer Ampulle eingesetzt werden. Die Anwendung ist aber nicht darauf beschränkt, sondern auch andere Wirkstofflösungen können damit in der Human- oder Tiermedizin appliziert werden, besonders, wenn eine kleine, leichte tragbare Pumpe erforderlich ist. Auch für den Transport von Perfusionsflüssigkeit von kontinuierlicher Mikrodialyse ist die vorliegende Erfindung geeignet. In technischen Systemen kann eine Pumpe mit einer erfindungsgemäßen Antriebsvorrichtung eingesetzt werden, um kleine Mengen zum Beispiel von Schmierstoffen oder Reagenzien in unterschiedlichsten Anwendungen zu dosieren.

Die Erfindung bezieht sich weiterhin auf ein Verfahren zum Dosieren eines flüssigen Produkts durch Verschieben eines Kolbens in einem das flüssige Produkt enthaltenden Behältnis mit Hilfe einer Antriebsvorrichtung, wobei das Behältnis begrenzende Seitenwände aufweist, die sich in Längsrichtung zu einem Auslass für das flüssige Produkt hin erstrecken und die Antriebsvorrichtung ein erstes, dem Auslass zugewandtes Segment, ein zweites von dem Auslass abgewandtes Segment und ein zwischen den zwei Segmenten angeordnetes und diese miteinander verbindendes Verbindungssegment umfasst, mit den Verfahrensschritten:
- Verklemmen des zweiten Segments zwischen den Seitenwänden durch eine aktive Formänderung des zweiten Segments,
- Lösen des ersten Segments von den Seitenwänden durch eine aktive Formänderung des ersten Segments,
- Vergrößern der Länge des Verbindungssegments, wobei sich das erste Segment in Richtung des Auslasses verschiebt,
- Verklemmen des ersten Segments zwischen den Seitenwänden durch eine aktive Formänderung des ersten Segments,
- Lösen des zweiten Segments von den Seitenwänden durch eine aktive Formänderung des zweiten Segments und
- Verkleinern der Länge des Verbindungssegments, wobei sich das zweite Segment in Richtung des Auslasses verschiebt.

Dabei werden die Verfahrensschritte in dieser Reihenfolge zyklisch durchgeführt, wobei nach dem letzten Verfahrensschritt der erste folgt. Die Ruheposition der Antriebsvorrichtung ist zum Beispiel nach der Durchführung des zweiten Verfahrensschritts erreicht, wenn das zweite Segment verklemmt ist, das erste Segment gelöst ist und das Verbindungssegment eine kleine Länge aufweist. Durch die in den Verfahrensschritten genannte Bewegungsfolge bewegt sich die Antriebsvorrichtung "raupenartig" auf den Auslass des Behältnisses zu und drückt den Kolben vorwärts und damit das flüssige Produkt aus dem Behältnis durch den Auslass heraus.

Die Erfindung wird nachstehend anhand der Zeichnung näher erläutert.
- Figur 1: zeigt eine Ampulle mit einem Kolben, in die eine erfindungsgemäße Antriebsvorrichtung eingesetzt ist,
- Figuren 2a und 2b: zeigen eine Ausführungsform eines ersten Segments einer erfindungsgemäßen Antriebsvorrichtung,
- Figur 3a und 3b: zeigen eine Ausführungsform eines zweiten Segments einer erfindungsgemäßen Antriebsvorrichtung und
- Figur 4a bis 4g: zeigen einen Bewegungsablauf einer erfindungsgemäßen Antriebsvorrichtung.

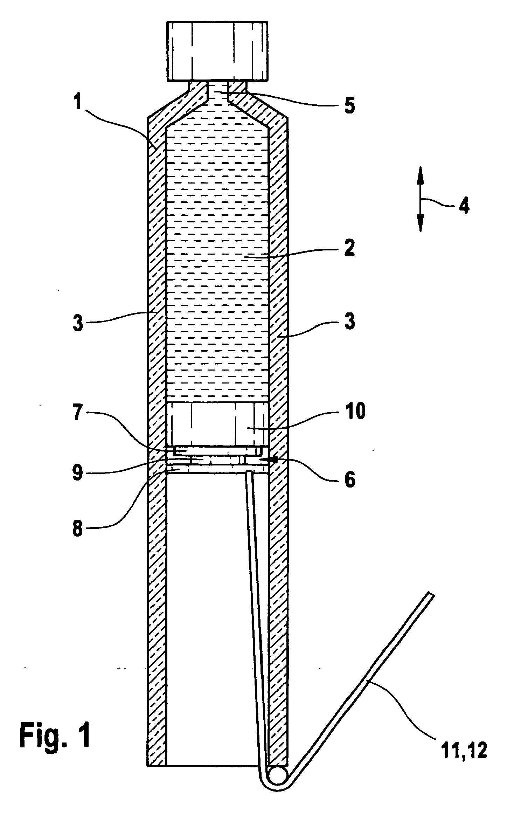

Figur 1 zeigt ein Behältnis mit einer erfindungsgemäßen Antriebsvorrichtung.

Das Behältnis 1 ist eine Ampulle, die ein flüssiges Produkt 2 (zum Beispiel Insulin) enthält. Das Behältnis 1 weist begrenzende Seitenwände 3 auf, die sich in Längsrichtung 4 zu einem Auslass 5 für das flüssige Produkt 2 hin erstrecken. Ein verschiebbarer Kolben 10 ist in dem Behältnis 1 angeordnet. Die erfindungsgemäße Antriebsvorrichtung 6 weist ein erstes Segment 7, ein zweites Segment 8 und ein Verbindungssegment 9 auf. Das Verbindungssegment 9 weist eine aktiv veränderbare Länge auf (Verbindungslänge), wobei diese Länge den Abstand der beiden Segmente 7, 8 voneinander bestimmt. Der Kolben 10 trennt die Antriebsvorrichtung 6 von dem flüssigen Produkt 2 und ist fest mit dem ersten Segment 7 der Antriebsvorrichtung 6 verbunden, so dass das Segment 7 zwar seine Form aktiv verändern kann, jedoch stets direkt an dem Kolben 10 anliegt. In Ruheposition ist die Antriebsvorrichtung 6 (mindestens ein Segment) in dem Behältnis 1 zwischen den Seitenwänden verklemmt und hält so den Kolben 10 in seiner Position. Die Signal- und Energieversorgung 11 der Antriebsvorrichtung 6 erfolgt über das Kabel 12.

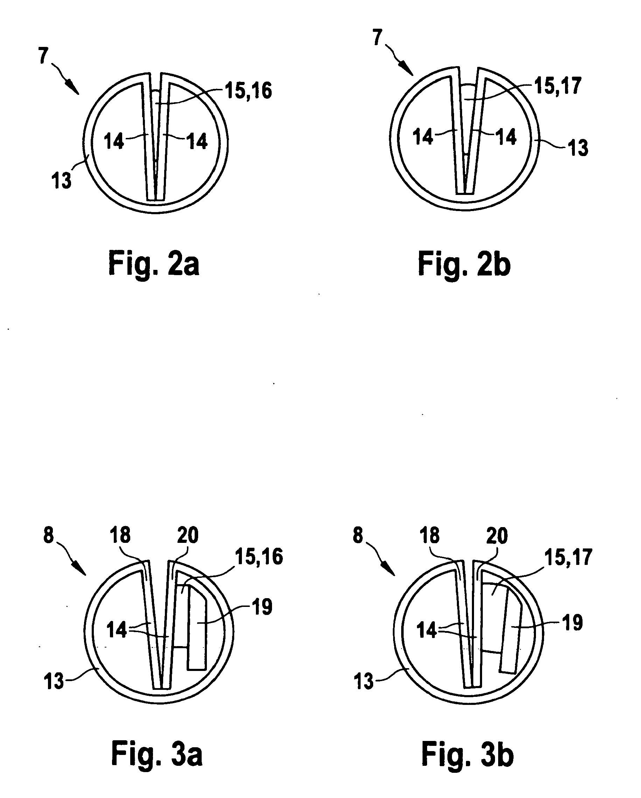

Figuren 2a und 2b zeigen eine Ausführungsform eines ersten Segments einer erfindungsgemäßen Antriebsvorrichtung.

Das erste Segment 7 ist als offener Ring 13 mit zwei in den Ring 13 hineinragenden Zungen 14 ausgebildet, wobei zwischen den beiden Zungen 14 ein erster Aktuator 15 angeordnet ist, zum Beispiel ein Paraffinaktuator. Die Dicke des Aktuators 15 ist aktiv veränderbar, zum Beispiel durch Erwärmen und Abkühlen des Aktuators 15.

Das in Figur 2a dargestellte erste Segment 7 zeigt den Aktuator 15, wenn er deaktiviert 16 ist. Das Segment 7 befindet sich in seiner Ruheposition mit einem ersten kleineren Ringdurchmesser. Mit diesem kleineren Ringdurchmesser wäre das Segment 7 von den Seitenwänden eines umgebenden Behältnisses gelöst und in diesem verschiebbar. Das erste Segment 7 der erfindungsgemäßen Antriebsvorrichtung hat eine aktiv veränderbare Form. Durch eine Aktivierung des Aktuators 15 wird eine solche Formänderung erreicht.

Das in Figur 2b dargestellte erste Segment 7 zeigt den Aktuator 15, wenn er aktiviert 17 ist. Das Segment 7 befindet sich in einer gespreizten Position mit einem zweiten größeren Ringdurchmesser, da der aktivierte Aktuator 17 durch seine größere Dicke die Zungen 14 auseinanderdrückt. Mit diesem größeren Ringdurchmesser wäre das Segment 7 zwischen den Seitenwänden eines umgebenden Behältnisses verklemmt und in diesem weitgehend unverschiebbar.

Um die Ruheposition (Figur 2a) des ersten Segments 7 wieder zu erreichen, wird der Aktuator 15 wieder deaktiviert.

Figuren 3a und 3b zeigen eine Ausführungsform eines zweiten Segments einer erfindungsgemäßen Antriebsvorrichtung.

Das zweite Segment 8 ist als offener Ring 13 mit zwei in den Ring 13 hineinragenden Zungen 14 ausgebildet. Die linke Zunge 18 und ein Widerlager 19, das neben der rechten Zunge 20 angeordnet ist, sind mit einer (nicht dargestellten - in der Bildebene liegenden) Grundplatte verbunden. Zwischen der rechten Zunge 20 und dem Widerlager 19 ist ein zweiter Aktuator 15 angeordnet, dessen Dicke aktiv (zum Beispiel durch Erhitzen oder Abkühlen) veränderbar ist.

Das in Figur 3a dargestellte zweite Segment 8 zeigt den Aktuator 15, wenn er deaktiviert 16 ist. Das Segment 8 befindet sich in seiner Ruheposition mit einem ersten größeren Ringdurchmesser. Mit dem größeren Ringdurchmesser wäre das Segment 8 zwischen den Seitenwänden eines umgebenden Behältnisses verklemmt und in diesem weitgehend unverschiebbar. Das zweite Segment 8 der erfindungsgemäßen Antriebsvorrichtung hat eine aktiv veränderbare Form. Durch eine Aktivierung des Aktuators 15 wird eine solche Formänderung erreicht.

Das in Figur 3b dargestellte zweite Segment 8 zeigt den Aktuator 15, wenn er aktiviert 17 ist. Das Segment 8 befindet sich in einer zusammengedrückten Position mit einem zweiten kleineren Ringdurchmesser, da der Aktuator 17, der sich am Widerlager 19 abstützt, durch seine Ausdehnung die rechte Zunge 20 zu der linken Zunge 18 hindrückt. Mit diesem kleineren Ringdurchmesser wäre das Segment 8 von den Seitenwänden eines umgebenden Behältnisses gelöst und in diesem verschiebbar.

Um die Ruheposition (Figur 3a) des zweiten Segments 8 wieder zu erreichen, wird der Aktuator 15 wieder deaktiviert.

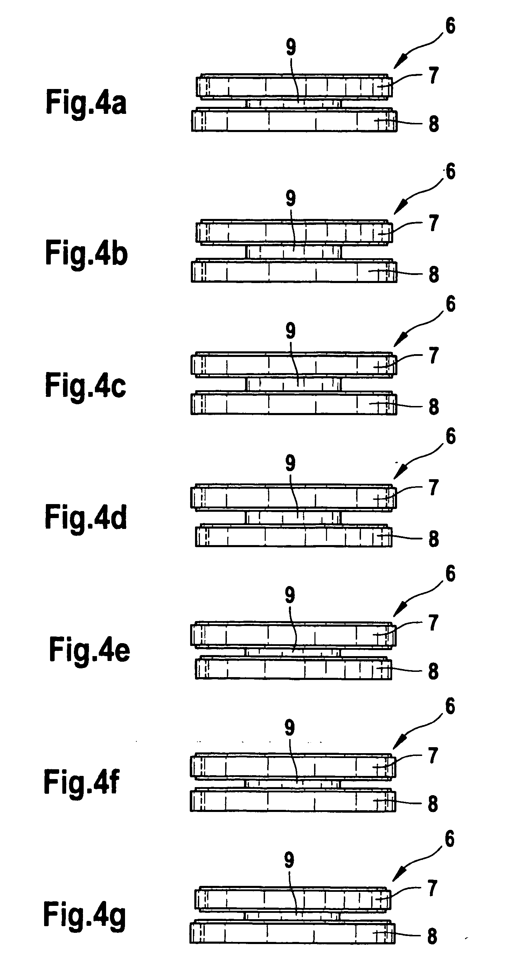

Figuren 4a bis 4g zeigen einen Bewegungsablauf einer erfindungsgemäßen Antriebsvorrichtung.

Der Bewegungsablauf zeigt, wie die zwei Segmente 7, 8 und das Verbindungssegment 9 zusammenwirken, um einen sicheren Vorschub des (nicht dargestellten) Kolbens in dem (nicht dargestellten) Behältnis zu gewährleisten. Der Bewegungsablauf ist in 6 Schritten dargestellt, wobei nach dem sechsten Schritt (Figur 4f) wieder der erste Schritt (Figur 4g) folgt.

Schritt 1 (Figur 4a) stellt die Ruheposition der Antriebsvorrichtung 6 dar. Alle drei Aktuatoren, die dem ersten Segment 7, dem Verbindungssegment 9 und dem zweiten Segment 8 zugeordnet sind, sind inaktiv. Im inaktiven Fall ist das erste Segment 7 klein, das Verbindungssegment 9 kurz und das zweite Segment 8 groß. Durch eine Aktivierung des jeweiligen Aktuators wird das erste Segment 7 groß, das Verbindungssegment 9 lang beziehungsweise das zweite Segment 8 klein. Der Zustand (aktiv oder inaktiv) des jeweiligen Aktuators in den verschiedenen Bewegungsschritten 1 bis 6 (Figuren 4a bis 4f) ist der folgenden Tabelle zu entnehmen:

| Bewegungsschritt | 1 | 2 | 3 | 4 | 5 | 6 |
|---|---|---|---|---|---|---|
| | (Fig.4a) | (Fig.4b) | (Fig.4c) | (Fig.4d) | (Fig.4e) | (Fig.4f) |
| Aktuator des ersten Segments | I | I | A | A | A | A |
| Aktuator des Verbindungssegments | I | A | A | A | I | I |
| Aktuator des zweiten Segments | I | I | I | A | A | I |

wobei I bedeutet, dass der Aktuator inaktiv und A bedeutet, dass der Aktuator aktiv ist.

Wenn ein Segment 7, 8 groß ist, ist es zwischen den Seitenwänden eines Behältnisses verklemmt, wenn es klein ist, ist es von den Seitenwänden des Behältnisses gelöst. In Schritt 1 (Fig. 4a) ist das zweite Segment 8 verklemmt und das erste Segment 7 gelöst. In Schritt 2 (Fig. 4b) wird das Verbindungssegment 9 lang und schiebt das erste Segment 7 nach oben. In Schritt 3 (Fig. 4c) sind beide Segmente 7, 8 verklemmt. In Schritt 4 (Fig. 4d) ist das erste Segment 7 verklemmt und das zweite Segment 8 gelöst. In Schritt 5 (Fig. 4e) wird das Verbindungssegment 9 kurz und zieht das zweite Segment 8 nach oben. In Schritt 6 (Fig. 4f) sind beide Segmente 7, 8 verklemmt. Dann folgt mit Schritt 1 (Fig. 4g) wieder die Ruheposition.

In der in Figuren 4a bis 4g dargestellten Ausführungsform besteht die erfindungsgemäße Antriebsvorrichtung für den Kolben eines Behältnisses (Ampulle) aus den zwei Segmenten 7, 8, die über einen Aktuator als Verbindungselement 9 miteinander verbunden sind. Das untere Segment enthält einen außenliegenden Spannring, der das Segment gegen die innere Wandung- der Ampulle verklemmt. Diese Verklemmung wird durch Zusammendrücken des Spannrings mit einem weiteren Aktuator erreicht. Das obere Segment 7 enthält ebenfalls einen außenliegenden, allerdings im Normalfall entspannten Spannring, der mit einem Aktuator gegen die innere Wandung der Ampulle verklemmt werden kann. Zu Beginn einer Bewegung wird der Aktuator des unteren Segments entspannt und damit das untere Segment gegenüber der Ampulle fixiert. Der Aktuator des oberen Segments ist ebenfalls entspannt und damit ist das obere Segment beweglich gegenüber der Ampulle.

Der Aktuator 9, der beide Segmente miteinander verbindet, wird aktiviert. Dadurch bewegt sich das obere Segment nach oben und nimmt den Kolben der Ampulle mit. Ist die Bewegung zu Ende, so wird der Aktuator am Spannring des oberen Segments 7 aktiviert und das Segment 7 gegen die Wandung der Ampulle verklemmt. Danach wird der Aktuator am Spannring des unteren Segments 8 aktiviert und die Verklemmung gelöst. Der Aktuator 9 zwischen beiden Segmenten 7, 8 wird deaktiviert und damit wird das untere Segment 8 nachgezogen. Der Aktuator am Spannring des unteren Segments 8 wird deaktiviert. Damit verklemmt sich das untere Segment 8 wieder gegen die Wandung der Ampulle. Der Aktuator am Spannring des oberen Segments 7 wird ebenfalls deaktiviert. Damit ist ein Bewegungszyklus abgeschlossen und alle Aktuatoren sind deaktiviert. Durch eine Vielzahl solcher Zyklen kann die Ampulle in gesteuerter Weise entleert werden.

Zur Entfernung der Antriebsvorrichtung aus der Ampulle wird der Aktuator des unteren Segments 8 aktiviert, der Aktuator des oberen Segments 7 bleibt deaktiviert. Dadurch ist die Verklemmung vollständig aufgehoben und der Antrieb kann an Verbindungskabeln oder einem Fangdraht aus der Ampulle gezogen werden.

### Bezugszeichenliste

- 1: Behältnis
- 2: flüssiges Produkt
- 3: Seitenwände
- 4: Längsrichtung
- 5: Auslass
- 6: Antriebsvorrichtung
- 7: erstes Segment
- 8: zweites Segment
- 9: Verbindungssegment
- 10: Kolben
- 11: Signal- und Energieversorgung
- 12: Kabel
- 13: offener Ring
- 14: Zungen
- 15: Aktuator
- 16: deaktivierter Aktuator
- 17: aktivierter Aktuator
- 18: linke Zunge
- 19: Widerlager
- 20: rechte Zunge

## Patentansprüche

1. Antriebsvorrichtung für einen Kolben (10) in einem ein flüssiges Produkt (2) enthaltenden Behältnis (1), wobei das Behältnis (1) begrenzende Seitenwände (3) aufweist, die sich in Längsrichtung (4) zu einem Auslass (5) für das flüssige Produkt (2) hin erstrecken, umfassend
• ein erstes, dem Auslass (5) zugewandtes Segment (7) mit einer aktiv veränderbaren Form, das in einer ersten Form zwischen den Seitenwänden (3) des Behältnisses (1) verklemmt ist und in einer zweiten Form von den Seitenwänden (3) weitgehend gelöst ist,
• ein zweites, von dem Auslass (5) abgewandtes Segment (8) mit einer aktiv veränderbaren Form, das in einer ersten Form zwischen den Seitenwänden (3) des Behältnisses (1) verklemmt ist und in einer zweiten Form von den Seitenwänden (3) weitgehend gelöst ist und
• ein Verbindungssegment (9), das zwischen dem ersten (7) und dem zweiten (8) Segment angeordnet ist und das erste (7) und zweite (8) Segment miteinander verbindet, wobei das Verbindungssegment (9) eine aktiv veränderbare Verbindungslänge aufweist,
**dadurch gekennzeichnet, dass** das erste und das zweite Segment (7, 8) erste und zweite Aktuatoren (15) zur aktiven Veränderung der Form des ersten beziehungsweise des zweiten Segments (7, 8) umfassen und dass die Aktuatoren Paraffinaktuatoren sind und die Antriebsvorrichtung Heizelemente zum Erwärmen der Paraffinaktuatoren umfasst.

2. Antriebsvorrichtung gemäß Anspruch 1, **dadurch gekennzeichnet, dass** das erste Segment (7) mit dem Kolben (10) fest verbunden ist oder einstückig mit dem Kolben (10) ausgebildet ist.

3. Antriebsvorrichtung gemäß einem der Ansprüche 1 oder 2, **dadurch gekennzeichnet, dass** das erste Segment (7) so ausgebildet ist, dass es bei inaktivem erstem Aktuator in der zweiten Form vorliegt und dass das zweite Segment (8) so ausgebildet ist, dass es bei inaktivem zweiten Aktuator in der ersten Form vorliegt.

4. Antriebsvorrichtung gemäß einem der Ansprüche 1 bis 3, **dadurch gekennzeichnet, dass** das Behältnis (1) eine Ampulle mit kreisförmigem Querschnitt ist und die ersten und zweiten Segmente (7, 8) Spannringe umfassen, die mit Hilfe jeweils eines Aktuators (15) zwischen einer inneren Wandung der Ampulle verklemmbar oder von dieser lösbar sind.

5. Antriebsvorrichtung gemäß Anspruch 4, **dadurch gekennzeichnet, dass** die Spannringe jeweils einen Ringdurchmesser aufweisen, der durch ein Aktivieren der Aktuatoren (15) veränderbar ist.

6. Antriebsvorrichtung gemäß einem der Ansprüche 1 bis 5, **dadurch gekennzeichnet, dass** das erste Segment (7) als offener Ring (13) mit zwei in den Ring (13) hineinragenden Zungen (14) ausgebildet ist, wobei zwischen den beiden Zungen (14) ein Aktuator (15) angeordnet ist, dessen Dicke aktiv veränderbar ist.

7. Antriebsvorrichtung gemäß einem der Ansprüche 1 bis 6, **dadurch gekennzeichnet, dass** das zweite Segment (8) als offener Ring (13) mit zwei in den Ring (13) hineinragenden Zungen (14, 18, 20) ausgebildet ist, wobei eine der Zungen (18) und ein neben der anderen Zunge (20) angeordnetes Widerlager (19) mit einer Grundplatte verbunden sind und zwischen der anderen Zunge (20) und dem Widerlager (19) ein Aktuator (15) angeordnet ist, dessen Dicke aktiv veränderbar ist.

8. Verfahren zum Dosieren eines flüssigen Produktes durch Verschieben eines Kolbens in einem das flüssige Produkt enthaltenden Behältnis mit Hilfe einer Antriebsvorrichtung gemäß einem der Ansprüche 1 bis 7, wobei das Behältnis begrenzende Seitenwände aufweist, die sich in Längsrichtung zu einem Auslass für das flüssige Produkt hin erstrecken und die Antriebsvorrichtung ein erstes, dem Auslass zugewandtes Segment, ein zweites, von dem Auslass abgewandtes Segment und ein zwischen den zwei Segmenten angeordnetes und diese miteinander verbindendes Verbindungssegment umfasst, **gekennzeichnet durch**
• Verklemmen des zweiten Segments zwischen den Seitenwänden **durch** eine aktive Formänderung des zweiten Segments,
• Lösen des ersten Segments von den Seitenwänden **durch** eine aktive Formänderung des ersten Segments,
• Vergrößern der Länge des Verbindungssegments, wobei sich das erste Segment in Richtung des Auslasses verschiebt,
• Verklemmen des ersten Segments zwischen den Seitenwänden **durch** eine aktive Formänderung des ersten Segments,
• Lösen des zweiten Segments von den Seitenwänden **durch** eine aktive Formänderung des zweiten Segments und
• Verkleinern der Länge des Verbindungssegments, wobei sich das zweite Segment in Richtung des Auslasses verschiebt.

9. Verwendung einer Antriebsvorrichtung gemäß einem der Ansprüche 1 bis 7 zum Dosieren eines flüssigen Produkts in einem Infusionssystem.

## Claims

1. Drive mechanism for a plunger (10) in a container (1) containing a liquid product (2), which container (1) has delimiting side walls (3) extending in the longitudinal direction (4) to an outlet (5) for the liquid product (2), comprising
• a first segment (7) with an actively variable shape facing the outlet (5), which is clamped between the side walls (3) of the container (1) in a first shape and largely released from the side walls (3) in a second shape,
• a second segment (8) with an actively variable shape remote from the outlet (5), which is clamped between the side walls (3) of the container (1) in a first shape and largely released from the side walls (3) in a second shape, and
• a connecting segment (9) which is disposed between the first (7) and the second (8) segment and connects the first (7) and second (8) segment to one another, and the connecting segment (9) has an actively variable connection length,
**characterised in that** the first and the second segment (7, 8) have first and second actuators (15) for actively varying the shape of the first respectively the second segment (7, 8), and the actuators are paraffin actuators and the drive mechanism has heating elements for heating the paraffin actuators.

2. Drive mechanism as claimed in claim 1, **characterised in that** the first segment (7) is fixedly connected to the plunger (10) or is made integrally with the plunger (10).

3. Drive mechanism as claimed in one of claims 1 or 2, **characterised in that** the first segment (7) is configured so that it assumes the second shape when the first actuator is inactive and the second segment (8) is configured so that it assumes the first shape when the second actuator is inactive.

4. Drive mechanism as claimed in one of claims 1 to 3, **characterised in that** the container (1) is an ampoule with a circular cross-section and the first and second segments (7, 8) are clamping rings which can be respectively clamped between an inner wall of the ampoule or released from it by means of an actuator (15).

5. Drive mechanism as claimed in claim 4, **characterised in that** the clamping rings respectively have an annular diameter which can be varied by activating the actuators (15).

6. Drive mechanism as claimed in one of claims 1 to 5, **characterised in that** the first segment (7) is an open ring (13) with two tongues (14) extending into the ring (13), and an actuator (15), the thickness of which can be actively varied, is disposed between the two tongues (14).

7. Drive mechanism as claimed in one of claims 1 to 6, **characterised in that** the second segment (8) is an open ring (13) with two tongues (14, 18, 20) extending into the ring (13), and one of the tongues (18) and a thrust bearing (19) disposed next to the other tongue (20) are connected to a base plate, and an actuator (15), the thickness of which can be actively varied, is disposed between the other tongue (20) and the thrust bearing (19).

8. Method of administering doses of a liquid product by pushing a plunger in a container containing the liquid product with the aid of a drive mechanism as claimed in one of claims 1 to 7, which container has delimiting side walls extending in the longitudinal direction to an outlet for the liquid product, and the drive mechanism comprises a first segment facing the outlet, a second segment remote from the outlet and a connecting segment disposed between the two segments and connecting them to one another, **characterised by**
• clamping the second segment between the side walls by actively changing the shape of the second segment,
• releasing the first segment from the side walls by actively changing the shape of the first segment,
• increasing the length of the connecting segment so that the first segment is pushed in the direction of the outlet,
• clamping the first segment between the side walls by actively changing the shape of the first segment,
• releasing the second segment from the side walls by actively changing the shape of the second segment and
• reducing the length of the connecting segment so that the second segment is pushed in the direction of the outlet.

9. Use of a drive mechanism as claimed in one of claims 1 to 7 for administering doses of a liquid product in an infusion system.

## Revendications

1. Dispositif d'entraînement pour un piston (10) situé dans un contenant (1) renfermant un produit liquide (2), le contenant (1) comportant des parois latérales de délimitation (3) qui s'étendent dans le sens longitudinal (4) vers une sortie (5) pour le produit liquide (2), comprenant
- un premier segment (7) tourné vers la sortie (5), avec une forme activement modifiable, lequel est, sous une première forme, coincé entre les parois latérales (3) du contenant (1) et est, sous une deuxième forme, largement détaché des parois latérales (3),
- un deuxième segment (8) opposé à la sortie (5), avec une forme activement modifiable, lequel est, sous une première forme, coincé entre les parois latérales (3) du contenant (1) et est, sous une deuxième forme, largement détaché des parois latérales (3) et
- un segment de liaison (9) qui est situé entre le premier segment (7) et le deuxième segment (8) et relie entre eux le premier segment (7) et le deuxième segment (8), le segment de liaison (9) présentant une longueur de liaison activement modifiable,
**caractérisé en ce que** les premier et deuxième segments (7, 8) comprennent un premier et un deuxième actionneurs (15) pour modifier activement la forme du premier respectivement du deuxième segment (7, 8) et **en ce que** les actionneurs sont des actionneurs à paraffine et le dispositif d'entraînement comprend des éléments de chauffage pour réchauffer les actionneurs à paraffine.

2. Dispositif d'entraînement selon la revendication 1, **caractérisé en ce que** le premier segment (7) est solidaire du piston (10) ou est réalisé d'une seule pièce avec le piston (10).

3. Dispositif d'entraînement selon l'une des revendications 1 ou 2, **caractérisé en ce que** le premier segment (7) est réalisé de manière telle qu'il se présente sous la deuxième forme lorsque le premier actionneur est inactif et **en ce que** le deuxième segment (8) est réalisé de manière telle qu'il se présente sous la première forme lorsque le deuxième actionneur est inactif.

4. Dispositif d'entraînement selon l'une des revendications 1 à 3, **caractérisé en ce que** le contenant (1) est une ampoule de section circulaire et les premier et deuxième segments (7, 8) comprennent des bagues de serrage qui, à l'aide de respectivement un actionneur (15), peuvent être coincés entre une paroi intérieure de l'ampoule ou en sont détachables.

5. Dispositif d'entraînement selon la revendication 4, **caractérisé en ce que** les bagues de serrage ont chacune un diamètre annulaire qui est modifiable par activation des actionneurs (15).

6. Dispositif d'entraînement selon l'une des revendications 1 à 5, **caractérisé en ce que** le premier segment (7) se présente sous la forme d'une bague ouverte (13) avec deux languettes (14) faisant saillie dans la bague (13), un actionneur (15) dont l'épaisseur est modifiable activement étant situé entre les deux languettes (14).

7. Dispositif d'entraînement selon l'une des revendications 1 à 6, **caractérisé en ce que** le deuxième segment (8) se présente sous la forme d'une bague ouverte (13) avec deux languettes (14, 18, 20) faisant saillie dans la bague (13), l'une des languettes (18) et une butée (19) situé à côté de l'autre languette (20) étant reliées à une plaque de base et un actionneur (15) dont l'épaisseur est modifiable activement étant situé entre l'autre languette (20) et la butée (19).

8. Procédé pour doser un produit liquide en déplaçant un piston dans un contenant renfermant le produit liquide à l'aide d'un dispositif d'entraînement selon l'une des revendications 1 à 7, le contenant comportant des parois latérales de délimitation qui s'étendent dans le sens longitudinal vers une sortie pour le produit liquide et le dispositif d'entraînement comprenant un premier segment tourné vers la sortie, un deuxième segment opposé à la sortie et un segment de liaison qui est situé entre les deux segments et les relie entre eux, **caractérisé par**
- le coinçage du deuxième segment entre les parois latérales par une modification active de la forme du deuxième segment,
- le détachement du premier segment des parois latérales par une modification active de la forme du premier segment,
- l'augmentation de la longueur du segment de liaison, le premier segment se déplaçant en direction de la sortie,
- le coinçage du premier segment entre les parois latérales par une modification active de la forme du premier segment,
- le détachement du deuxième segment des parois latérales par une modification active de la forme du deuxième segment et
- la réduction de la longueur du segment de liaison, le deuxième segment se déplaçant en direction de la sortie.

9. Utilisation d'un dispositif d'entraînement selon l'une des revendications 1 à 7 pour doser un produit liquide dans un système de perfusion.
